(19) Europäisches Patentamt
European Patent Office
Office européen des brevets

(11) Veröffentlichungsnummer: **0 030 703**
**B1**

(12) # EUROPÄISCHE PATENTSCHRIFT

(45) Veröffentlichungstag der Patentschrift: **21.03.84**

(21) Anmeldenummer: **80107746.2**

(22) Anmeldetag: **09.12.80**

(51) Int. Cl.³: **C 07 D417/04**// C09B57/02, D06L3/12 ,(C07D417/04, 285/12, 311/12),(C07D417/04, 285/12, 311/16),(C07D417/04, 277/64, 311/12),(C07D417/04, 277/64, 311/16)

(54) **Verfahren zur Herstellung von Cumarinverbindungen.**

(30) Priorität: **14.12.79 DE 2950291**

(43) Veröffentlichungstag der Anmeldung: **24.06.81 Patentblatt 81/25**

(45) Bekanntmachung des Hinweises auf die Patenterteilung: **21.03.84 Patentblatt 84/12**

(84) Benannte Vertragsstaaten: **BE CH DE FR GB LI NL**

(56) Entgegenhaltungen:
**EP-A- 0 011 839**
**AT-B- 225 661**
**CH-A- 582 696**
**DE-A- 2 065 076**
**DE-A- 2 344 834**
**DE-A- 2 553 294**
**DE-A- 2 710 285**
**DE-A- 2 807 761**
**DE-B- 1 262 206**
**DE-B- 1 670 999**
**DE-B- 1 942 926**

(73) Patentinhaber: **BASF Aktiengesellschaft**
**Carl-Bosch-Strasse 38**
**D-6700 Ludwigshafen (DE)**

(72) Erfinder: **Hagen, Helmut, Dr.**
**Max-Slevogt-Strasse 17E**
**D-6710 Frankental (DE)**
Erfinder: **Kohler, Rolf-Dieter, Dr.**
**Amselweg 3**
**D-6803 Edingen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 030 703 B1

Verfahren zur Herstellung von Cumarinverbindungen

Die Erfindung betrifft ein Verfahren zur Herstellung von Cumarinverbindungen durch Umsetzung von Brenztraubensäurealkylestern mit Salicylaldehyden und anschließende Cyclisierung in Gegenwart basischer Verbindungen oder durch Umsetzung der Enolate von Brenztraubensäurealkylestern mit Salicylaldehyden.

Die Herstellung von 3-Phenylcumarin durch Umsetzung von Salicylaldehyd mit Benzylcyanid ist bekannt (Elderfield, Heterocyclic Compounds, Band 2 (1951), Seiten 174-176). US-A 3 338 784 beschreibt die Herstellung von 3-Benzthiazol-2-yl-cumarin durch Umsetzung von Cumarin-3-carbonsäurechlorid mit o-Aminothiophenol. In entsprechender Weise kann die 3-Benzimidazolylverbindung durch Umsetzung des Cumarin-3-carbonsäuremethylesters mit o-Phenylendiamin und Polyphosphorsäure hergestellt werden. Das Verfahren ist im Hinblick auf einfache und wirtschaftliche Arbeitsweise sowie Ausbeute und Reinheit des Endstoffs unbefriedigend.

Die DE-A1-2 710 285 beschreibt die Umsetzung von heterocyclischen Produkten, z. B. auch 2-Carbalkoxymethyl-1,3-benzoxazole und -benzothiazole, in Gegenwart organischer Lösungsmittel und starker Säuren mit Salicylaldehyden zu Cumarinfarbstoffen, die in 3-Stellung des Cumarinteiles einen heterocyclischen Rest tragen. Auch z. B. mit Alkoxy-, Phenoxy, mono- oder disubstituierter Aminogruppe oder einem heterocyclischen Rest substituierte Salicylaldehyde können verwendet werden. In den Beispielen werden nur 2-Cyanmethyl- und keine 2-Carbalkoxymethyl-heterocyclen veranschaulicht.

Die DE-A-2 065 076 zeigt eine ähnliche Umsetzung von 2-Hydroxyaldehyden, die in 4-Stellung eine mono- oder disubstituierte Aminogruppe tragen, mit Oxazolyl-2-essigsäure-derivaten zu 3-Oxazolylcumarinen.

Es wurde nun gefunden, daß man Cumarinverbindungen der Formel (I)

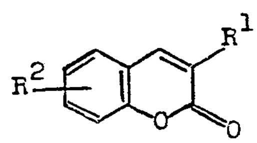

(I)

worin $R^1$ einen gegebenenfalls durch Alkylgruppen mit 1 bis 4 Kohlenstoffatomen substituierten 1,3,4-Thiadiazol-2-ylrest oder 1,3-Benzthiazol-2-ylrest bedeutet, $R^2$ ein Wasserstoffatom, oder den Rest

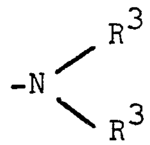

bezeichnet, die einzelnen Reste Rest $R^3$ gleich oder verschieden sein können und jeweils für einen Alkylrest mit 1 bis 7 Kohlenstoffatomen oder einen Aralkylrest mit 7 bis 12 Kohlenstoffatomen stehen oder beide Reste $R^3$ zusammen mit dem benachbarten Stickstoffatom Glieder eines Morpholinorestes bedeuten, vorteilhaft erhält, wenn man

a) Brenztraubensäurealkylester der Formel (II)

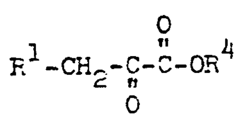

(II)

worin $R^1$ die vorgenannte Bedeutung besitzt und $R^4$ einen aliphatischen Rest bedeutet, bei einer Temperatur von 80 bis 160 °C in einem ersten Schritt mit Salicylaldehyden der Formel (III)

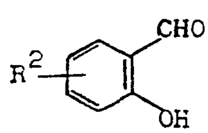

(III)

worin $R^2$ die vorgenannte Bedeutung besitzt, in Gegenwart von Säuren als Katalysator umsetzt und die so erhaltenen 4-(o-Hydroxyphenyl)-2oxo-buten-(3,4)-säureester der Formel (IV)

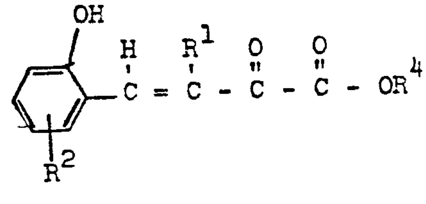

(IV)

worin $R^1$, $R^2$ und $R^4$ dir vorgenannte Bedeutung besitzen, in einem zweiten Schritt in Gegenwart von basischen Verbindungen und in Gegenwart von unter den Reaktionsbedingungen inerten Lösungsmitteln umsetzt, oder

b) Enolate der Brenztraubensäurealkylester (II) mit Salicylaldehyden (III) bei einer Temperatur von 80 bis 160 °C und in Gegenwart von unter den Reaktionsbedingungen inerten Lösungsmitteln umsetzt.

Die Umsetzung kann für den Fall der Verwendung von 5-Methyl-1,3,4-thiadiazol-2-yl-brenztraubensäureäthylester und Salicylaldehyd durch die folgenden Formeln wiedergegeben werden :

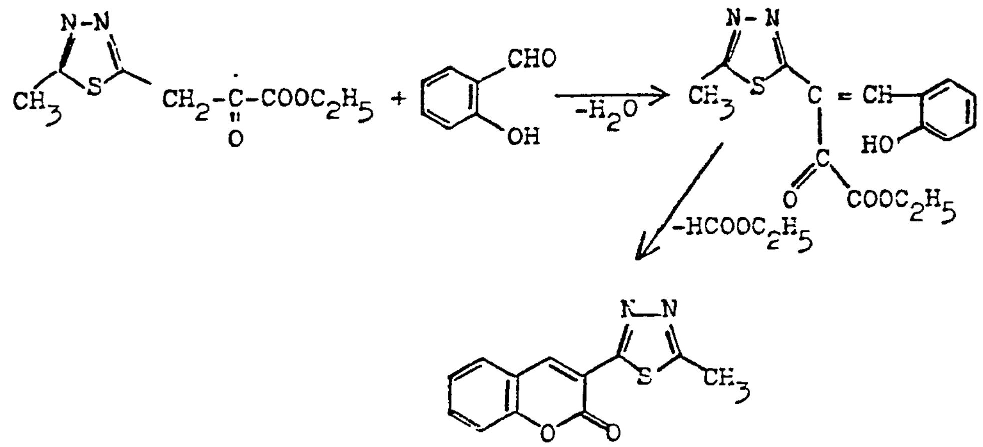


Im Vergleich zu dem bekannten Verfahren liefert das Verfahren nach der Erfindung die Cumarinverbindungen auf einfacherem und wirtschaftlichem Wege und in besserer Ausbeute und Reinheit. Alle diese vorteilhaften Ergebnisse des erfindungsgemäßen Verfahrens sind überraschend. Es konnte auch nicht vorhergesehen werden, daß der Ringschluß im 2. Schritt in Gegenwart basischer Verbindungen unter Eliminierung eines Ameisensäurebruchstücks glatt verläuft.

Der Ausgangsstoff (II) oder sein Enolat kann mit dem Ausgangsstoff (III) in stöchiometrischer Menge oder im Überschuß, vorzugsweise in einem Verhältnis von 1 bis 4 Mol Ausgangsstoff III je Mol Ausgangsstoff II oder Enolat, umgesetzt werden. Bevorzugte Ausgangsstoffe (II) und Enolate der Ausgangsstoffe (II) und dementsprechend bevorzugte Stoffe (IV) sind solche, in deren Formel $R^4$ einen Alkylrest mit 1 bis 4 Kohlenstoffatomen bedeutet.

Die Enolate der Ausgangsstoffe (II) können in bekannter Weise, z. B. durch Umsetzung der Ausgangsstoffe (II) mit Alkalimetallalkoholaten oder Alkalimetall in organischen Lösungsmitteln wie Benzol, oder mit Erdalkaliacetaten, -hydroxiden, -sulfaten oder mit Metallchloriden wie Eisen-III-chlorid, Chrom-III-chlorid, oder mit Alkoholaten mehrwertiger Metalle wie Aluminium, Eisen, Titan, Zirkon, Zinn, erhalten werden. Bezüglich der Herstellung wird auf Houben-Weyl, Methoden der Organischen Chemie, Band 6/2, Seiten 53 bis 62 verwiesen. Zweckmäßige Enolate sind solche der Formel (V)

$$R^1 - CH = \underset{OZ}{C} - \overset{O}{\overset{\|}{C}} - OR^4 \qquad \text{(V)}$$

worin $R^1$ und $R^4$ die vorgenannte allgemeine und bevorzugte Bedeutung besitzen und Z ein Alkaliatom, ein äquivalentes Erdalkaliatom, insbesondere Magnesium oder Strontium, oder ein Äquivalent von Kupfer, Nickel, Eisen, Kobalt, Mangan, Chrom, Aluminium, Titan, Zirkon, Zinn, bezeichnet. Bevorzugte Enolate sind Alkalienolate, insbesondere das Natriumenolat oder Kaliumenolat.

So kommen beispielsweise folgende Brenztraubensäurealkylester als Ausgangsstoffe (II) und entsprechend die folgenden Enolate in Betracht : β-1,3,4-Thiadiazol-2-yl- und β-1,3-Benzthiazolyl-(2)-brenztraubensäuremethylester ; entsprechende Äthyl-, Propyl-, Isopropyl-, Butyl-, Isobutyl-, sek.-Butyl-, tert.-Butyl-ester ; das Natrium- und Kaliumenolat vorgenannter Brenztraubensäureester.

Beispielsweise sind folgende Salicylaldehyde als Ausgangsstoffe (III) geeignet : Salicylaldehyd ; in 2-, 3-, 4- oder 5-Stellung durch die

Dimethylamino-, Diäthylamino-, Dipropylamino-, Diisopropylamino-, Dibutylamino-, Diisobutylamino-, Di-sek.-butylamino-, di-tert.-butylamino-, Dibenzylamino-, N-Morpholinogruppe substituiertes Salicylaldehyd.

Die Umsetzung wird bei einer Temperatur von 80 bis 160 °C, in der 1. Stufe der Verfahrensweise a) vorzugsweise von 80 bis 130 °C, in der 2. Stufe der Verfahrensweise a) vorzugsweise von 100 bis 160 °C, bei der Verfahrensweise b) vorzugsweise von 80 bis 150 °C, drucklos oder zweckmäßig unter Druck, kontinuierlich durchgeführt. Man verwendet unter den Reaktionsbedingungen inerte Lösungsmittel für die 2. Stufe der Reaktion a) und für die Reaktion b) und zweckmäßig für die 1. Stufe der Reaktion a). Als

Lösungsmittel kommen z. B. in Frage : aromatische Kohlenwasserstoffe, z. B. Toluol, Äthylbenzol, o-, m-, p-Xylol, Isopropylbenzol, Methylnaphthalin ; Alkanole und Cycloalkanole wie Äthanol, Methanol, n-Butanol, Isobutanol, tert.-Butanol, Glykol, Glycerin, n-Propanol, Isopropanol, Amylalkohol, Cyclohexanol, 2-Methyl-4-pentanol, Äthylenglykolmonoäthyläther, 2-Äthylhexanol, Methylglykol, n-Hexanol, Isohexylalkohol, Isoheptylalkohol, n-Heptanol, Äthylbutanol, Nonylalkohol, Dodecylalkohol, Methylcyclohexanol, Diacetonalkohol, insbesondere solche mit 1 bis 4 Kohlenstoffatomen ; Dimethylformamid ; und entsprechende Gemische. Zweckmäßig verwendet man das Lösungsmittel in einer Menge von 100 bis 10 000 Gewichtsprozent, vorzugsweise von 200 bis 1 500 Gewichtsprozent bezogen auf Ausgangsstoff (II).

Die Umsetzung wird in der 1. Stufe von a) in Gegenwart von Säure als Katalysator, vorteilhaft mit einer Menge von 0,01 bis 0,2, insbesondere von 0,02 bis 0,1 Äquivalenten Säure, bezogen auf 1 Mol Ausgangsstoff (II), durchgeführt. Es können zweckmäßig Lewis-Säuren oder organische Säuren verwendet werden. Anstelle einbasischer Säuren können auch äquivalente Mengen mehrbasischer Säuren zur Anwendung gelangen. Beispielsweise sind folgende Säuren geeignet : aliphatische Carbonsäuren wie Chloressigsäure, Dichloressigsäure, Trichloressigsäure, Ameisensäure, Essigsäure, Propionsäure, Buttersäure, Isobuttersäure, Glykolsäure, Milchsäure, Brenztraubensäure, Weinsäure, Zitronensäure, β-Oxybuttersäure, Caprylsäure, Trimethylessigsäure, α- bzw. β-Chlorpropionsäure, Isovaleriansäure, Valeriansäure ; aliphatischaromatische, araliphatische, aromatische Carbonsäuren wie Benzoesäure, o-, m- bzw. p-Oxybenzoesäure, Phenylpropionsäure, o-, m- bzw. p-Chlorbenzoesäure, 2,3,4-, 2,4,5-, 2,4,6-, 3,4,5-Trimethylbenzoesäure, Phenylessigsäure. Unter Lewis-Säuren werden hier elektrophile Stoffe mit unvollständiger Elektronenkonfiguration verstanden, die ein Elektronenpaar einer Base aufnehmen können. Bezüglich der Definition von Lewis-Säuren wird auf Houben-Weyl, Methoden der Organischen Chemie, Band 4/2, Seite 6, und Rodd, Chemistry of Carbon Compounds, Band IA, Seite 103 (Elsevier Publ. Co. N.Y. 1951) verwiesen. Es kommen zweckmäßig als Lewis-Säuren Halogenide von Metallen der 2. bis 6. Gruppe des Periodischen Systems wie Zink-, Bor-, Aluminium-, Zinn-, Titan-, Antimon-, Wismut-, Molybdän-, Wolfram-chlorid, Aluminiumbromid und Bortrifluorid in Frage. Die Lewis-Säure kann man ebenfalls in Gestalt ihrer Komplexe, z. B. von Bortrifluorid-ätherat, -dihydrat ; -äthylalkoholat und anderen -alkoholaten ; Fluorborsäure, Borfluoridessigsäure, -diessigsäure, -phosphorsäure ; Bortrichlorid-Komplexverbindungen mit Phosphortrichlorid und Phosphoroxychlorid verwenden.

Die erste Stufe der Reaktion a) kann wie folgt durchgeführt werden : Ein Gemisch von Katalysator, Ausgangsstoff (II) und (III), gegebenenfalls zusammen mit Lösungsmitteln, wird während 0,5 bis 6 Stunden bei der Reaktionstemperatur gehalten. Aus dem Reaktionsgemisch wird der Stoff (IV) in üblicher Weise, zweckmäßig durch Ausfällen mit einem geeigneten Lösungsmittel, z. B. Essigester, und Filtration abgetrennt.

Die Umsetzung in der zweiten Stufe der Reaktion a) wird in Gegenwart einer basischen Verbindung, vorteilhaft in einer Menge von 1 bis 8, vorzugsweise von 1,5 bis 6 Äquivalenten basischer Verbindung, bezogen auf ein Mol Ausgangsstoff II, durchgeführt. Als basische Verbindungen werden zweckmäßig tertiäre Amine, Erdalkaliverbindungen, Ammoniumverbindungen, tertiäre Phosphine und insbesondere Alkaliverbindungen sowie entsprechende Gemische verwendet. Bevorzugt sind Alkoholate, insbesondere Alkalialkoholate ; unter den Alkoholaten sind Alkanolate vorteilhaft. Es kommen z. B. als basische Verbindungen in Frage : Kaliumhydroxid, Natriumhydroxid, Lithiumhydroxid, Calciumhydroxid, Calciumoxid, Bariumoxid, Magnesiumhydroxid, Magnesiumoxid, Bariumhydroxid, Magnesiumacetat, Natriumformiat, Natriumacetat, Natriumpropionat, Natriumbutyrat, Natriumisobutyrat, Kaliumformiat, Kaliumacetat, Kaliumpropionat, Kaliumbutyrat, Kaliumisobutyrat ; Trimethylamin, Triäthylamin, Tripropylamin, Triisopropylamin, Tributylamin, Triisobutylamin, Tri-sek.-butylamin, Tri-tert.-butylamin, Tribenzylamin, Tricyclohexylamin, Triamylamin, Trihexylamin, N,N-Dimethylanilin, N,N-Diäthylanilin, N,N-Dipropylanilin, N,N-Dimethyltoluidin, N,N-Diäthyloluidin, N,N-Dipropyltoluidin, N,N-Dimethyl-p-aminopyridin, N,N-Diäthyl-p-aminopyridin, N,N-Dipropyl-p-aminopyridin, N,N-Dimethylaminoäthanol, N,N-Diäthylaminoäthanol, N,N-Dipropylaminoäthanol, N-Methylpyrrolidon, N-Äthylpyrrolidon, N-Methylpiperidin, N-Äthylpiperidin, N-Methylpyrrolidin, N-Äthylpyrrolidin, N-Methylimidazol, N-Äthylimidazol, N-Methylpyrrol, N-Äthylpyrrol, N-Methylmorpholin, N-Äthylmorpholin, N-Methylhexamethylenimin, N-Äthylhexamethylenimin, Pyridin, Chinolin, α-Picolin, β-Picolin, γ-Picolin, Isochinolin, Pyrimidin, Acridin, N,N,N',N'-Tetramethyläthylendiamin, N,N,N',N'-Tetraäthyläthylendiamin, Chinoxalin, Chinazolin, N-Propyldiisopropylamin, N,N-Dimethylcyclohexylamin, 2,6-Lutidin, 2,4-Lutidin, Trifurfuralamin, Triäthylendiamin ; bevorzugt Natriummethylat, Natriumäthylat, Natriumpropylat, Natriumisopropylat, Natriumbutylat, Natriumisobutylat, Natrium-sek.-butylat, Natrium-tert.-butylat, Natriumäthylenglykolat, Natriumpropylen-(1,2)-glykolat, Natriumpropylen-(1,3)-glykolat, Natriumdiäthylenglykolat, Natriumtriäthylenglykolat, Natriumdipropylen-(1,2)-glykolat, Kaliummethylat, Kaliumäthylat, Kalium-n-propylat, Kaliumisopropylat, Kalium-n-butylat, Kaliumisobutylat, Kalium-sek.-butylat, Kalium-tert.-butylat, Kaliumäthylenglykolat, Kaliumpropylen-(1,2)-glykolat, Kaliumpropylen-(1,3)-glykolat, Kaliumdiäthylenglykolat, Kaliumtriäthylenglykolat, Kaliumdipropylen-(1,2)-glykolat ; besonders bevorzugt sind vorgenannte Alkanolate mit 1 bis 4 Kohlenstoffatomen.

Die Reaktion in der 2. Stufe der Verfahrensweise a) kann wie folgt durchgeführt werden : Ein Gemisch von Ausgangsstoff (IV), basische Verbindung und Lösungsmittel wird während 1 bis 6 Stunden bei der Reaktionstemperatur gehalten. Dann wird der Endstoff aus dem Gemisch in üblicher Weise, z. B. durch Filtration, abgetrennt.

Die Reaktion bei der Verfahrensweise b) kann wie folgt durchgeführt werden : Ein Gemisch von

Enolat des Ausgangsstoffs (II), Ausgangsstoff (III) und Lösungsmittel wird während 0,5 bis 4 Stunden bei der Reaktionstemperatur gehalten. Dann wird aus dem Reaktionsgemisch der Endstoff in üblicher Weise, z. B. durch Filtration, abgetrennt.

Die nach dem Verfahren der Erfindung herstellbaren Endstoffe (I) sind wertvolle Ausgangsstoffe für die Herstellung von Farbstoffen, Schädlingsbekämpfungsmitteln und Pharmazeutika. Sie sind Fluoreszenfarbstoffe und optische Aufheller, insbesondere für Textilfasern und Textilgewebe, z. B. aus Baumwolle oder Polyesterfasern, Tuschen und Tinten. Bezüglich der Verwendung wird auf die genannte Veröffentlichungen und Ullmanns Encyklopädie der rechnischen Chemie, 4. Band (3. Auflage), Seiten 562 bis 565 verwiesen.

Die in den folgenden Beispielen aufgeführten Teile bedeuten Gewichtsteile.

Beispiel 1

a) (Herstellung von Stoff IV) Ein Gemisch aus 21,4 Teilen β-(5-Methyl-1,3,4-thiadiazol-2-yl)-brenztraubensäureäthylester, 18 Teilen Salicylaldehyd und 0,5 Teilen Zinkchlorid wird 2 Stunden bei 100 °C gerührt. Nach Abkühlen auf 20 °C gibt man unter Rühren 150 Teile Methanol zu und saugt den ausgefallenen Feststoff ab. Man erhält 19 Teile 2-[2'-(2"-Hydroxyphenyl)-1'-äthoxyoxalyl-vinyl]-5-methyl-1,3,4-thiadiazol (60 % der Theorie) vom Fp 202 °C (Toluol).

b) Zu einer Lösung von 79,5 Teilen 2-[2'-(2"-Hydroxyphenyl)-1'-äthoxyoxalyl-vinyl]-5-methyl-1,3,4-thiadiazol in 250 Teilen Methylglykol gibt man portionsweise 20,5 Teile Natriummethylat und rührt das Gemisch eine Stunde bei 130 °C. Dann wird das Gemisch auf 20 °C gekühlt und der ausgefallene Kristallbrei abgesaugt. Nach Waschen mit Äthylalkohol und Äther erhält man 52 Teile 3-(5'-Methyl-1',3',4'-thiadiazol-2'-yl)-cumarin (85 % der Theorie) vom Fp 238 °C.

Beispiel 2

a) (Herstellung von Stoff IV) Ein Gemisch aus 25 Teilen β-(1,3-Benzthiazol-2-yl)-brenztraubensäureäthylester, 36 Teilen Salicylaldehyd und ein Teil Zinkchlorid wird 2 Stunden auf 100 °C erwärmt. Dann kühlt man die Reaktionslösung auf 22 °C ab und versetzt mit 150 Teilen Essigester. Der ausgefallene Feststoff wird abgesaugt und getrocknet. Man erhält 25 Teile 2-[2'-(2"-Hydroxyphenyl)-1'-äthoxyoxalyl-vinyl]-1,3-benzthiazol (70 % der Theorie) vom Fp 198 °C.

b) Zu einer Lösung von 35 Teilen 2-[2'-(2"-Hydroxyphenyl)-1'-äthoxyoxalyl-vinyl]-1,3-benzthiazol in 150 Teilen Äthylenglykol gibt man 8,1 Teile Natriummethylat. Das Gemisch wird eine Stunde bei 130 °C gerührt, anschließend auf 20 °C gekühlt und vom ausgefallenen Feststoff abgesaugt. Nach Waschen mit Methanol und Trocknen erhält man 16 Teile 3-(1',3'-Benzthiazol-2'-yl)-cumarin (57 % der Theorie) vom Fp 216 °C.

Beispiel 3

Ein Gemisch aus 12,6 Teilen β-(5-Methyl-1,3,4-thiadiazol-2-yl)-brenztraubensäureäthylester-kaliumenolat und 10,5 Teilen 4-Morpholino-salicylaldehyd wird in 100 Teilen Methylglykol 3 Stunden auf 120 °C erhitzt. Anschließend kühlt man das Gemisch auf 20 °C, saugt den ausgefallenen Feststoff ab und wäscht ihn mit Methanol nach. Man erhält 7 Teile 3-(5'-Methyl-1',3',4'-thiadiazol-2'-yl)-7-morpholinocumarin (44 % der Theorie) vom Zersetzungspunkt 305 bis 310 °C (Methylglykol).

Beispiel 4

12,5 Teile β-(5-Methyl-1,3,4-thiadiazol-2-yl)-brenztraubensäureäthylester-kaliumenolat werden mit 16 Teilen 4-Dibenzylamino-salicylaldehyd in 100 Teilen Methylglykol 3 Stunden bei 120 °C gerührt. Nach Zugabe von 100 Teilen Methanol wird der ausgefallene Feststoff abgesaugt. Man erhält 12 Teile 3-(5'-Methyl-1',3',4'-thiadiazol-2'-yl)-7-dibenzylaminocumarin (54 % der Theorie) vom Fp 224 °C (Methylglykol).

Beispiel 5

Eine Lösung aus 14,3 Teilen β-(1,3-Benzthiazol-2-yl)-brenztraubensäureäthylester-kaliumenolat und 16 Teilen 4-Dibenzylamino-salicylaldehyd wird in 100 Teilen Methylglykol 3 Stunden bei 120 °C gerührt. Das Lösungsmittel wird dann im Vakuum entfernt. Der Rückstand wird mit 2n-Natronlauge aufgeschlämmt, abgesaugt und mit Wasser gewaschen. Man erhält 12 Teile 3-(1',3'-Benzthiazol-2'-yl)-7-dibenzylamino-cumarin (52 % der Theorie) vom Fp 183 °C (Methylglykol).

**Anspruch**

Verfahren zur Herstellung von Cumarinverbindungen der Formel (I)

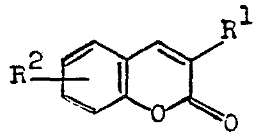

(I)

worin $R^1$ einen gegebenenfalls durch Alkylgruppen mit 1 bis 4 Kohlenstoffatomen substituierten 1,3,4-Thiadiazol-2-ylrest oder 1,3-Benzthiazol-2-ylrest bedeutet, $R^2$ ein Wasserstoffatom oder den Rest

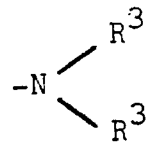

bezeichnet, die einzelnen Reste $R^3$ gleich oder verschieden sein können und jeweils für einen Alkylrest mit 1 bis 7 Kohlenstoffatomen oder einen Aralkylrest mit 7 bis 12 Kohlenstoffatomen stehen oder beide Rest $R^3$ zusammen mit dem benachbarten Stickstoffatom Glieder eines Morpholinorestes bedeuten, dadurch gekennzeichnet, daß man

a) Brenztraubensäurealkylester der Formel (II)

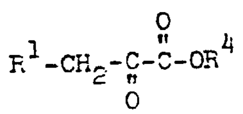

(II)

worin $R^1$ die vorgenannte Bedeutung besitzt und $R^4$ einen aliphatischen Rest bedeutet, bei einer Temperatur von 80 bis 160 °C, in einem ersten Schritt mit Salicylaldehyden der Formel (III)

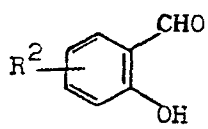

(III)

worin $R^2$ die vorgenannte Bedeutung besitzt, in Gegenwart von Säure als Katalysator umsetzt und die so erhaltenen 4-(o-Hydroxyphenyl)-2-oxo-buten-(3,4)-säureester der Formel (IV)

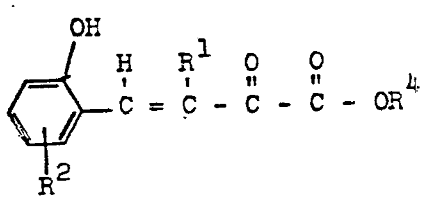

(IV)

worin $R^1$, $R^2$ und $R^4$ die vorgenannte Bedeutung besitzen, in einem zweiten Schritt in Gegenwart von basischen Verbindungen und in Gegenwart von unter den Reaktionsbedingungen inerten Lösungsmitteln umsetzt, oder

b) Enolate der Brenztraubensäurealkylester (II) mit Salicylaldehyden (III) bei einer Temperatur von 80 bis 160 °C und in Gegenwart von unter den Reaktionsbedingungen inerten Lösungsmitteln umsetzt.

**Claim**

A process for the preparation of coumarin compounds of the formula (I)

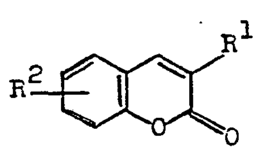

(I)

where $R^1$ is 1,3,4-thiadiazol-2-yl or 1,3-benzothiazol-2-yl optionally substituted by alkyl of 1 to 4 carbon atoms, $R^2$ is hydrogen or

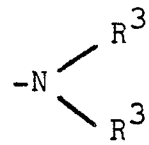

the individual $R^3$ is being identical or different and eachdenoting alkyl of 1 to 7 carbon atoms or aralkyl of 7 to 12 carbon atoms, or the two $R^{3}$'s together with the adjacent nitrogen atom denoting a morpholino radical, wherein

a) an alkyl pyruvate of the formula (II)

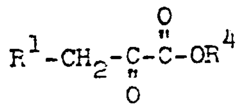

(II)

where $R^1$ has the above meanings and $R^4$ is an aliphatic radical, is reacted, in a first step, at 80 to 160 °C with a salicylaldehyde of the formula (III)

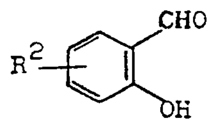

(III)

where $R^2$ has the above meaning, in the presence of an acid as catalyst, and the resulting 4-(o-hydroxyphenyl)-2-oxobut-3,4-enoic acid ester of the formula (IV)

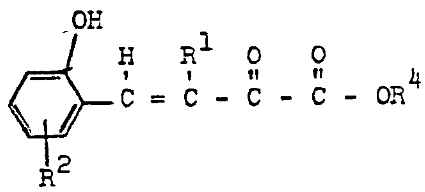

(IV)

where $R^1$, $R^2$ and $R^4$ have the above meanings, is reacted, in a second step, in the presence of basic compounds and in the presence of solvents which are inert under the reaction conditions, or

b) an enolate of the alkyl pyruvate (II) is reacted with a salicylaldehyde (III) at 80 to 160 °C in the presence of solvents are inert under the reaction conditions.

**Revendication**

Procédé de préparation de dérivés de la coumarine de la formule (I)

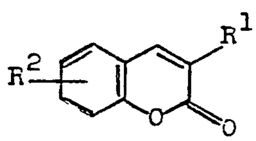

(I)

dans laquelle $R^1$ désigne un groupe 1,3-benzothiazol-2-yle ou 1,3,4-thiadiazol-2-yle éventuellement substitué par des radicaux alkyle en $C_1$ à $C_4$ et $R^2$ un atome d'hydrogène ou un groupe

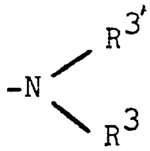

dont les restes $R^3$, qui peuvent être identiques ou différents, représentent chacun un radical alkyle en $C_1$ à $C_7$ ou un groupe aralkyle en $C_7$ à $C_{12}$ ou forment avec l'atome d'azote adjacent des chaînons d'un groupe morpholino, caractérisé en ce que

a) on fait réagir un ester d'alkyle de l'acide pyruvique, de la formule (II)

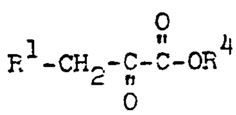

(II)

dans laquelle $R^1$ possède la signification définie et $R^4$ désigne un reste aliphatique, dans un premier stade, en présence d'un acide comme catalyseur et à une température comprise entre 80 et 160 °C, avec un aldéhyde salicylique de la formule (III)

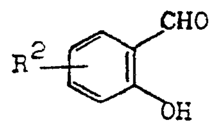

(III)

dans laquelle $R^2$ possède la signification définie, et ensuite, dans un deuxième stade, les esters de l'acide 4-(o-hydroxyphényl)-2-oxo-butén-(3,4)-oïque formés, de la formule (IV)

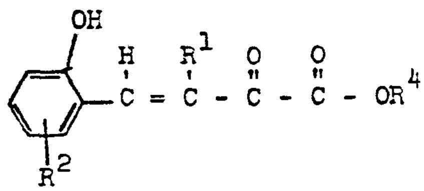

(IV)

dans laquelle $R^1$, $R^2$ et $R^4$ possèdent les significations définies, en présence de composés basiques dans des solvants inertes dans les conditions réactionnelles ; ou

b) on fait réagir un énolate de l'ester d'alkyle de l'acide pyruvique (II) en présence de solvants inertes dans les conditions réactionnelles et à une température de 80 à 160 °C avec un aldéhyde salicylique (III).